(19) Europäisches Patentamt
European Patent Office
Office européen des brevets

(11) Veröffentlichungsnummer: **0 450 292 A2**

(12) **EUROPÄISCHE PATENTANMELDUNG**

(21) Anmeldenummer: **91102250.7**

(51) Int. Cl.5: **G01N 33/00**

(22) Anmeldetag: **18.02.91**

(30) Priorität: **31.03.90 DE 4010425**

(43) Veröffentlichungstag der Anmeldung:
**09.10.91 Patentblatt 91/41**

(84) Benannte Vertragsstaaten:
**DE FR GB IT SE**

(71) Anmelder: **Degussa Aktiengesellschaft**
**Weissfrauenstrasse 9**
**W-6000 Frankfurt am Main 1(DE)**

(72) Erfinder: **Brand, Reinhold, Dr.**
**Gustav-Adolf-Strasse 25**
**W-6450 Hanau 1(DE)**
Erfinder: **Engler, Bernd H, Dr.**
**Treuener Strasse 2**
**W-6450 Hanau 9(DE)**
Erfinder: **Honnen, Wolfgang, Dr.**
**Am Heinichenberg 38**
**W-6454 Bruchköbel(DE)**
Erfinder: **Koberstein, Edgar, Dr.**
**Wolfskernstrasse 8**
**W-8755 Alzenau(DE)**

(54) **Vorrichtung und Verfahren zur kontinuierlichen Messung der Ammoniakkonzentration in Gasen im ppm-Bereich.**

(57) Eine Vorrichtung zur kontinuierlichen Messung der Ammoniakkonzentration in Gasen im ppm-Bereich hat ein säulenförmiges Absorptionsgefäß (1) mit halbkugelförmigem Boden (4). Letzter hat einen Überlauf (3) und einen Auslauf (5). Oberhalb (4) und unterhalb eines in Säule (1) angeordneten Siebbodens (6) mündet das Gaseinleitungsrohr (7, 8). Auf dem Siebboden ruht eine Füllkörperpackung (10), welche (1) erfüllt. (1) setzt sich nach oben in einen Kühlteil (11) fort. Dieser hat eine Kühlzone (15) und einen darüber befindlichen Gassammelraum (16) mit Saugstutzen (13). Ein beidseitig zackenkronenartig endigendes Rohr (14) verläuft von (16) aus gekühlt abwärts vor die Füllkörperpackung. Das flüssige Absorptionsmittel wird durch einen Seitenstutzen (18) in den Gassammelraum eingegeben. Zur Messung wäscht man einen bestimmten Volumenstrom des Gases im Gegenstrom mit dem Absorptionsmittel, ermittelt in einem Teil davon den $NH_3$-Gehalt, mißt die Volumenströme des abfließenden Absorptionsmittels und des von $NH_3$ befreiten und getrockneten Gases und berechnet die Ammoniakkonzentration.

EP 0 450 292 A2

Die Erfindung betrifft eine neuartige Vorrichtung zur kontinuierlichen Messung der Ammoniakkonzentration in Gasen, z. B. Rauchgasen, im ppm-Bereich und ein Verfahren für die Messung, das sich mit der genannten Vorrichtung durchführen läßt.

Die quantitative Bestimmung der Ammoniakkonzentration in gasförmigen Medien hat durch die Einführung sekundärer Entstickungsmaßnahmen bei Feuerungsanlagen einen wichtigen Stellenwert bekommen. In den meisten Fällen wird zur Stickoxidminderung die Methode der selektiven katalytischen Reduktion mit Ammoniak als Reduktionsmittel angewandt. Zur Steuerung solcher Anlagen und zur Überwachung der vorgeschriebenen Emissionswerte ist eine kontinuierliche Ammoniak-Messung wünschenswert.

Zur Zeit sind Geräte zur Messung der $NH_3$-Konzentration bekannt, die auf verschiedenen Verfahrensprinzipien beruhen und für unterschiedliche Meßbereiche geeignet sind.

Hierbei kann unterschieden werden zwischen Verfahren, die $NH_3$ direkt in der Gasphase detektieren und solchen, bei denen $NH_3$ chemisch gebunden und dann in Lösung bestimmt wird.

Verfahren, mit denen die $NH_3$-Konzentration in der Gasphase bestimmt werden kann, basieren meist auf der Photometrie des $NH_3$ im IR-Bereich. Hierzu wird über eine Gassonde Probegas entnommen und in die Küvette des Photometers geleitet. Der kleinste Meßbereich der meisten im Handel erhältlichen Geräte liegt bei 0 - 300 ppm.

Der speziell bei der Überwachung von Denox-Anlagen interessante Bereich zwischen 0 und 10 ppm wird daher von solchen Geräten nicht mehr zuverlässig abgedeckt. Mittlerweile sind zwar Laserphotometer im Handel, mit denen in situ $NH_3$-Konzentrationen um 1 ppm nachgewiesen werden können. Diese Geräte sind jedoch außerordentlich kostspielig, benötigen eine lange Absorptionsstrecke und weisen hohe Fehlerschwankungen auf. Ein weiteres Verfahren zur Bestimmung der $NH_3$-Konzentration in der Gasphase beruht auf der Verbrennung des $NH_3$ an einem Katalysator und der Messung der Wärmetönung der Reaktion. Zuverlässig funktioniert dieses Verfahren nur bei hoheren $NH_3$-Konzentrationen und bei Abwesenheit weiterer oxidierbarer Stoffe [z. B. CO, Kohlenwasserstoffe).

Verfahren, bei denen $NH_3$ aus der Gasphase chemisch gebunden und dann in Lösung bestimmt wird, werden häufiger angewendet, da sie empfindlicher sind. Sie haben jedoch den Nachteil, zeitaufwendiger und meist diskontinuierlich zu arbeiten.

Des weiteren finden oft kolorimetrische Verfahren Anwendung, bei denen die Lichtabsorption eines bei einer Farbreaktion aus $NH_3$ und anderen Reagenzien entstehenden Farbstoffes zur Konzentrationsbestimmung ausgenutzt wird.

Auch die ionensensitive Potentiometrie und die konventionelle naßchemische Bestimmung des $NH_3$ per Säure-Base-Titration nach einer Kjeldahl-Destillation sind vielfach benutzte Verfahren. Sie sind aber diskontinuierlich und bedürfen meist eines beachtlichen Zeitaufwands bis zum fertigen Analysenergebnis. Weitere, weniger häufig angewandte Verfahren benutzen die Photoionisation des $NH_3$ oder auch elektrochemische Sensoren. Hierbei wird jedoch der gewünschte Meßbereich zwischen 0 und 10 ppm nicht erreicht.

Gegenstand der Erfindung ist eine Vorrichtung zur kontinuierlichen Messung der Ammoniakkonzentration in Gasen im ppm-Bereich. Die Vorrichtung ist gekennzeichnet durch ein säulenförmiges Absorptionsgefäß 1 mit halbkugelförmig ausgebauchtem Boden 4 und einem an dessen tiefster Stelle befindlichen Ablaufstutzen 5, einem umittelbar oberhalb des Bodens 4 seitlich angesetzten Überlaufrohr 3, einem oberhalb des Bodens 4 und unterhalb eines im Gefäß 1 befestigten Siebbodens 6 seitlich in das Gefäß 1 geführten und in diesem mittig nach oben gebogenen und mit einer Spülleitung 9 versehenen Einleitungsrohr 7, 8 für das zu messende Gas, einer auf dem Siebboden aufliegenden und Gefäß 1 im wesentlichen ausfüllenden Füllkörperpackung 10 sowie einem das Absorptionsgefäß 1 nach oben fortsetzenden Kühlteil 11 mit einer Kühlzone 15, einem darüber befindlichen Gassammelraum 16 mit verschließbarem Saugstutzen 13, einem seitlich in den Raum 16 mündenden Stutzen 18 für die Zugabe des flüssigen Absorptionsmittels und einem unterhalb der Kühlzone 15 mit dem Mantel des Kühlteils 11 dicht verbundenen, durch die Kühlzone 15 verlaufenden und mit seiner Mündung in den Gassammelraum 16 ragenden, an beiden Enden zackenkronenartig ausgebildeten Innenrohr 14.

Eine bevorzugte Ausführungsform der erfindungsgemäßen Vorrichtung sieht vor, daß Absorptionsgefäß und Kühlteil als getrennte. miteinander gegebenenfalls über Schliffe verbindbare Einzelstücke ausgebildet sind.

Ein weiterer Gegenstand der Erfindung ist ein Verfahren zur kontinuierlichen Messung der Ammoniakkonzentration in Gasen im ppm-Bereich, welches unter Einsatz der vorstehend gekennzeichneten Vorrichtung ausgeübt werden kann. Es ist dadurch gekennzeichnet, daß man einen bestimmten Volumenstrom des zu messenden Gases kontinuierlich im Gegenstrom mit einem flüssigen Absorptionsmittel in Berührung bringt, wenigstens einen Teil des ammoniakbeladenen Absorptionsmittels in einem Ammoniakkanalysator quantitativ auf seine $NH_3$-Konzentration untersucht, den Volumenstrom des gesamten abfließenden Absorptionsmittels und den Volumenstrom des von Ammoniak befreiten und getrockneten Gases mißt und die

$NH_3$-Konzentration nach der Formel

$$c^G_{NH_3} = \frac{c^L_{NH_3} \cdot \dot{V}_{Abs} \cdot 10^6 \cdot V_{M,NH_3}}{\dot{V}^G \cdot M_{NH_3}} \quad [ppm],$$

berechnet, wobei

| | | | |
|---|---|---|---|
| $c^G_{NH_3}$ | = | Konzentration $NH_3$ im Gas | [ppm bez. auf trocken] |
| $c^L_{NH_3}$ | = | Konzentration $NH_3$ in der Absorptionslösung | [$g/dm^3$] |
| $\dot{V}_{Abs.}$ | = | Volumenstrom der abfließenden Absorptionslösung | [$dm^3/h$] |
| $\dot{V}_G$ | = | Volumenstrom des durch das Absorptionsgefäß gesaugte zu analysierende Gas | [$dm^3/h$ in Normzustand bez. auf trocken] |
| $\dot{V}_{M,NH_3}$ | = | Molvolumen von gasförmigem Ammoniak | [$\frac{dm^3}{Mol}$ im Normzustand] |
| $M_{NH_3}$ | = | Molmasse von Ammoniak | [$\frac{g}{Mol}$] |

Eine bevorzugte Ausführungsform der erfindungsgemäßen Vorrichtung ist in Figur 1 dargestellt und hat folgenden Aufbau:
Die Vorrichtung besteht aus einem zylindrischen, säulenförmigen Absorptionsgefäß 1 aus Glas von 14,4 mm Innendurchmesser und 225 mm Länge, welches am oberen Ende eine Normschliffhülse 2 (NS 29 nach DIN 12242 Teil 1) trägt. Gefäß 1 ist mit einem zunächst horizontal aus ihm austretenden und dann L-förmig nach unten gebogenen und am Ende mit einer Schliffhülse (NS 14) zum Aufstecken eines Sammelkolbens für das nicht zur Messung kommende $NH_3$-haltige Absorptionsmittel versehenen seitlichen Überlaufrohr 3 oberhalb eines nach unten halbkugelförmig ausgebauchten Bodens 4 ausgerüstet. Am tiefsten Punkt des Bodens 4 ist ein Ablaßstutzen 5 zur Absaugung der zur analytischen Messung kommenden $NH_3$-beladenen Waschflüssigkeit angebracht.

Ca. 170 mm unterhalb der Normschliffhülse 2 befindet sich ein Siebboden 6 mit runden Löchern von ca, 2 mm Durchmesser. Ca. 11 mm unterhalb des Siebbodens mündet zum Einbringen des Gases ein Anschlußstutzen 7 radial in den Glaszylinder 1. Im Inneren des Glaszylinders bildet ein L-förmig nach oben gebogenes und nach einer kurzen Strecke endigendes Glasrohr 8 die Fortsetzung dieses Anschlußstutzens. Durch diese Anordnung des Glasrohrs 8 wird das zu analysierende Gas unmittelbar auf die Unterseite des Siebbodens gerichtet, um ein Austrocknen der Glaswandungen des Absorptionsgefäßes durch das gegebenenfalls heiße Probegas zu vermeiden.

Am äußeren Ende des Stutzens 7 ist in etwa 24 mm Abstand von dem Mantel des Glaszylinders ein Kugelschliff befestigt. In der Mitte des Stutzens 7 ist ein Spülstutzen 9 angebracht, der nach oben weist. Durch diesen Stutzen kann zum Spülen des Gaseintrittsstutzens 7 von Zeit zu Zeit etwas Waschflüssigkeit eingebracht werden.

Eine Füllkörperpackung 10, z. B. aus Kugeln, Ringen, Hohlzylindern bzw. üblichen Misch- und Gasaustauschelementen aus Keramik, Glas oder Kunststoff, füllt die Säule 1 oberhalb des Siebbodens 6 bis etwa zum Ansatz der Schliffhülse 2.

Auf der Schliffhülse 2 des Absorptionsgefäßes 1 ist ein Kühlteil 11 aufgesetzt. Der Kühlaufsatz steht über den Schliffkern (.NS 29) 12 mit der Schliffhülse 2 in Verbindung.

Der Kühlaufsatz 11 besteht aus einem zum Teil doppelwandigen zylinderförmigen Glasrohr von 26 mm Durchmesser und ca. 90 mm Länge. Das Glasrohr endet oben in einem Einweghahn 13, welcher mit einer Leitung zu einer Gas-Saugpumpe in Verbindung steht, Das im Absorptionsgefäß 1 von $NH_3$ befreite Gas passiert den Kühlaufsatz 11 von unten nach oben durch ein Innenrohr 14 mit 8 mm Innendurchmesser, welches in einem oberhalb einer Kühlzone 15 befindlichen Gassammelraum 16 endet. Das Innenrohr 14 ist an beiden Enden als Zackenkrone ausgebildet.

Die Kühlzone 15 ist nach Art eines Liebigkühlers konstruiert und mit einer üblichen Kühlmittelführung 17 ausgerüstet. In den Gassammelraum 16 mündet seitlich ein Stutzen 18 für die Zugabe der Absorptionsflüssigkeit. Sie läuft über die obere Zackenkrone in das Innenrohr und tropft danach über die untere Zackenkrone auf die Füllkörperpackung 10. Die Zackenkronen bewirken eine möglichst gleichmäßige Beaufschlagung der Füllkörper mit der Absorptionsflüssigkeit. Das Innenrohr kann durch den Doppelmantel, je nach Bedarf, mit fließendem Wasser gekühlt werden.

Beim Betrieb der Vorrichtung stellt sich nach kurzer Anlaufzeit von wenigen Sekunden eine stationäre $NH_3$-Konzentration in der abfließenden Waschflüssigkeit ein, aus der sich mit Hilfe der folgenden Formel die $NH_3$-Konzentration im zu analysierenden Gas berechnen läßt:

$$c^{G}_{NH_3} = \frac{c^{L}_{NH_3} \cdot \dot{V}_{Abs} \cdot 10^6 \cdot \dot{V}_{M,NH_3}}{\dot{V}^{G} \cdot M_{NH_3}} \quad [ppm],$$

wobei

$c^G_{NH_3}$ = Konzentration $NH_3$ im Gas [ppm bez. auf trocken]

$c^L_{NH_3}$ = Konzentration $NH_3$ in der Absorptionslösung [$g/dm^3$]

$\dot{V}_{Abs.}$ = Volumenstrom der abfließenden Absorptionslösung [$dm^3/h$]

$\dot{V}_G$ = Volumenstrom des durch das Absorptionsgefäß gesaugten zu analysierenden Gases [$dm^3/h$ in Normzustand bez. auf trocken]

$\dot{V}_{M,NH_3}$ = Molvolumen von gasförmigem Ammoniak [$\frac{dm^3}{Mol}$ im Normzustand]

$M_{NH_3}$ = Molmasse von Ammoniak [$\frac{g}{Mol}$]

Zur Bedienung des $NH_3$-Analysators wird zunächst seine Basislinie durch Ansaugen von frischer Waschflüssigkeit einjustiert. Dann erfolgt eine Kalibrierung mit einer exakt eingestellten Lösung von $NH_4Cl$ in Waschflüssigkeit, Anschließend saugt man die sich am Boden des Absorptionsgefäßes sammelnde Waschflüssigkeit ab und beginnt mit dem Durchleiten des zu analysierenden Gases. Nachdem sich auf dem $NH_3$-Analysator ein konstanter Wert eingestellt hat, berechnet man nach der vorgegebenen Formel die Konzentration.

Der Verfahrensgang unter Einsatz der neuartigen Vorrichtung zur kontinuierlichen Bestimmung der Ammoniakkonzentration in Gasen im ppm-Bereich wird nachfolgend anhand von Figur 2 erläutert:

Das zu untersuchende Gas wird mit Hilfe einer Gaspumpe 22 durch eine Sonde und eine Gasleitung abgesaugt. Der abgesaugte Volumenstrom kann mit einem Nadelventil, das im Bypass zur Gaspumpe geschaltet ist, geregelt werden. Er liegt, zum Beispiel im Falle des Abgases aus einer großtechnischen Feuerungsanlage, zwischen 5 und 200 l/h und beträgt vorzugsweise 15 - 45 l/h i. Norm. Das abgesaugte Gas wird über einen mit Kugelschliff versehenen Stutzen 7,8 in die Absorptionsvorrichtung eingeleitet. Hier strömt das Gas von unten durch eine Säule 1 aus Glas mit einer Füllkörperpackung 10 und wird zur Absorption des Ammoniaks mit der Absorptionsflüssigkeit, die von oben auf die Füllkörperpackung aufgegeben wird, im Gegenstrom in Kontakt gebracht.

Als Absorptionsflüssigkeit können Lösungen von Säuren, wie $H_2SO_4$, $H_3BO_3$, HCl, zum Beispiel als 1 molare wäßrige Lösungen verwendet werden. Der Volumenstrom der Absorptionsflüssigkeit liegt beim oben genannten Anwendungsfall zwischen 0,03 und 1,0 l/h, vorzugsweise bei 0,1 bis 0,5 l/h.

Die Absorption des Ammoniaks geschieht folgendermaßen:

Die Absorptionsflüssigkeit wird mit Hilfe einer Schlauchpumpe 19a aus einem Vorratsgefäß 20 auf die Füllkörperpackung 10 gepumpt.

Das flüssige Absorptionsmittel tropft durch die Füllkörperpackung, belädt sich mit dem $NH_3$ und fällt auf den halbkugelförmig ausgebildeten Boden 4 der Absorptionssäule 1, wo es sich sammelt. Von dort fließt ein Teil der Lösung durch einen Überlauf 3 ab. Der andere Teil der Lösung wird mittels einer Schlauchpumpe 19b abgesaugt und in einen Ammoniakkanalysator 21 gepumpt. Der Ammoniakkanalysator basiert auf dem Prinzip der Gasdiffusion von Ammoniak durch eine Membran mit anschließender konduktometrischer Ammoniakbestimmung (verwendet wurde ein Ammoniakkanalysator des Typs Wescan 360 der Firma Gamma Analysentechnik).

Das von Ammoniak befreite Gas tritt oberhalb der Packung 10 durch einen Kühler 11, passiert über

Hahn 13 von Figur 1 die Pumpe 22, einen mit einem Trockenmittel gefüllten Trockenturm 23 und schließlich eine Gasuhr 24.

Die Erfindung wird an einem praktischen Anwendungsbeispiel weiter erläutert.

Beispiel

Die $NH_3$-Bestimmungsapparatur wurde zur Messung des $HN_3$-Schlupfes an einer Anlage zur Testung von SCR-Katalysatoren mit Rauchgas verwendet. Die $NH_3$-Konzentration wurde zusätzlich jeweils naßchemisch bestimmt

$$(= c^{G}_{NH_3}, \text{ soll}).$$

Das Rauchgas hatte folgende Zusammensetzung.

| | |
|---|---|
| $O_2$ = | 4,5 Vol.% tr. |
| $H_2O$ = | 10 Vol.% tr. |
| $CO_2$ = | 11 Vol.% tr. |
| $SO_2$ = | 70 ppm tr. |
| $NO_x$ = | 200 ppm tr. |
| $N_2$ = | Rest |

| Beispiel | Waschflüssigkeit | $\dot{V}_{Abs.}$ | $\dot{V}_G$ | $c^G_{NH_3}$ | $c^G_{NH_3}$, soll |
|---|---|---|---|---|---|
| 1 | 1 m $H_2SO_4$ | 0,236 | 36,0 | 198,3 | 200 |
| 2 | 1 m $H_2SO_4$ | 0,238 | 58,0 | 19,6 | 20 |
| 3 | 1 m $H_2SO_4$ | 0,236 | 36,9 | 20,2 | 20 |
| 4 | 1 m $H_2SO_4$ | 0,236 | 37,0 | 10,3 | 10 |
| 5 | 1 m $H_2SO_4$ | 0,236 | 37,0 | 4,7 | 5 |
| 6 | 1 m $H_2SO_4$ | 0,236 | 37,0 | 1,9 | 2 |
| 7 | 1 m $H_3BO_3$ | 0,240 | 40,0 | 19,8 | 20 |
| 8 | 1 m $H_3BO_3$ | 0,237 | 40,0 | 4,9 | 5 |

## Patentansprüche

1. Vorrichtung zur kontinuierlichen Messung der Ammoniakkonzentration in Gasen im ppm-Bereich, **gekennzeichnet durch** ein säulenförmiges Absorptionsgefäß (1) mit halbkugelförmig ausgebauchtem Boden (4) und einem an dessen tiefster Stelle befindlichen Ablaufstutzen (5), einem unmittelbar oberhalb des Bodens (4) seitlich angesetzten Uberlaufrohr (3), einem oberhalb des Bodens (4) und unterhalb eines im Gefäß (1) befestigten Siebbodens (6) seitlich in das Gefäß (1) geführten und in diesem mittig nach oben gebogenen und mit einer Spülleitung (9) versehenen Einleitungsrohr (7, 8) für das zu messende Gas, einer auf dem Siebboden aufliegenden und Gefäß (1) im wesentlichen ausfüllenden Füllkörperpackung (10) sowie einem das Absorptionsgefäß (1) nach oben fortsetzenden Kühlteil (11) mit einer Kühlzone (15), einem darüber befindlichen Gassammelraum (16) mit verschließbarem Saugstutzen (13), einem seitlich in den Raum (16) mündenden Stutzen (18) für die Zugabe des flüssigen Absorptionsmittels und einem unterhalb der Kühlzone (15) mit dem Mantel des Kühlteils (11) dicht verbundenen, durch die Kühlzone (15) verlaufenden und mit seiner Mündung in den Gassammelraum (16) ragenden, an beiden Enden zackenkronenartig ausgebildeten Innenrohr (14).

2. Vorrichtung nach Anspruch 1,
**dadurch gekennzeichnet,**
daß Absorbtionsgefäß und Kühlteil als getrennte, miteinander gegebenenfalls über Schliffe verbindbare Einzelstücke ausgebildet sind.

3. Verfahren zur kontinuierlichen Messung der Ammoniakkonzentration in Gasen im ppm-Bereich insbesondere unter Einsatz der Vorrichtung nach vorstehenden Ansprüchen,
**dadurch gekennzeichnet,**
daß man einen bestimmten Volumenstrom des zu messenden Gases kontinuierlich im Gegenstrom mit einem flüssigen Absorptionsmittel in Berührung bringt, wenigstens einen Teil des ammoniakbeladenen Absorptionsmittels in einem Ammoniakanalysator quantitativ auf seine $NH_3$-Konzentration untersucht, den Volumenstrom des gesamten abfließenden Absorptionsmittels und den Volumenstrom des von Ammoniak befreiten und getrockneten Gases mißt und die $NH_3$-Konzentration nach der Formel

$$c^G_{NH_3} = \frac{c^L_{NH_3} \cdot \dot{V}_{Abs} \cdot 10^6 \cdot V_{M,NH_3}}{\dot{V}^G \cdot M_{NH_3}} \quad [ppm],$$

berechnet, wobei

$c^G_{NH_3}$ = Konzentration $NH_3$ im Gas [ppm bez. auf trocken]

$c^L_{NH_3}$ = Konzentration $NH_3$ in der Absorptionslösung [g/dm³]

$\dot{V}_{Abs.}$ = Volumenstrom der abfließenden Absorptionslösung [dm³/h]

$\dot{V}_G$ = Volumenstrom des durch das Absorptionsgefäß gesaugten, zu analysierenden Gases. [dm³/h in Normzustand bez. auf trocken]

$\dot{V}_{M,NH_3}$ = Molvolumen von gasförmigem Ammoniak [$\frac{dm^3}{Mol}$ im Normzustand]

$M_{NH_3}$ = Molmasse von Ammoniak [$\frac{g}{Mol}$]

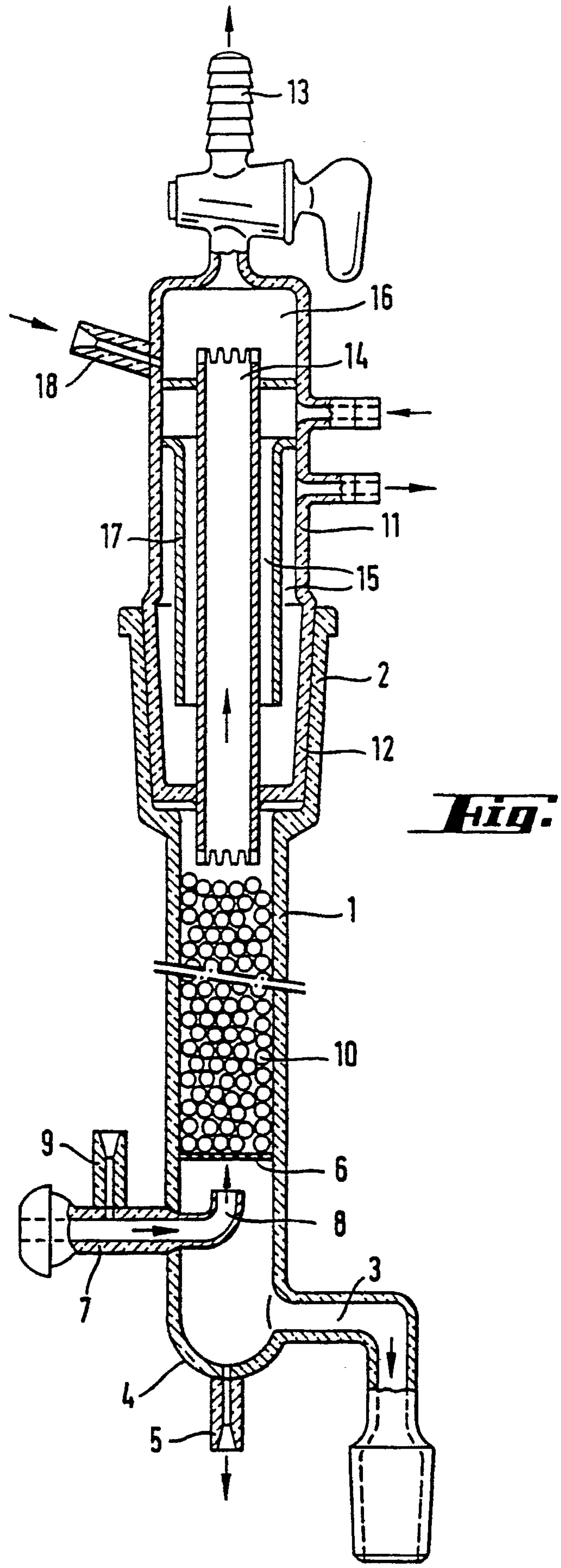


Fig. 1

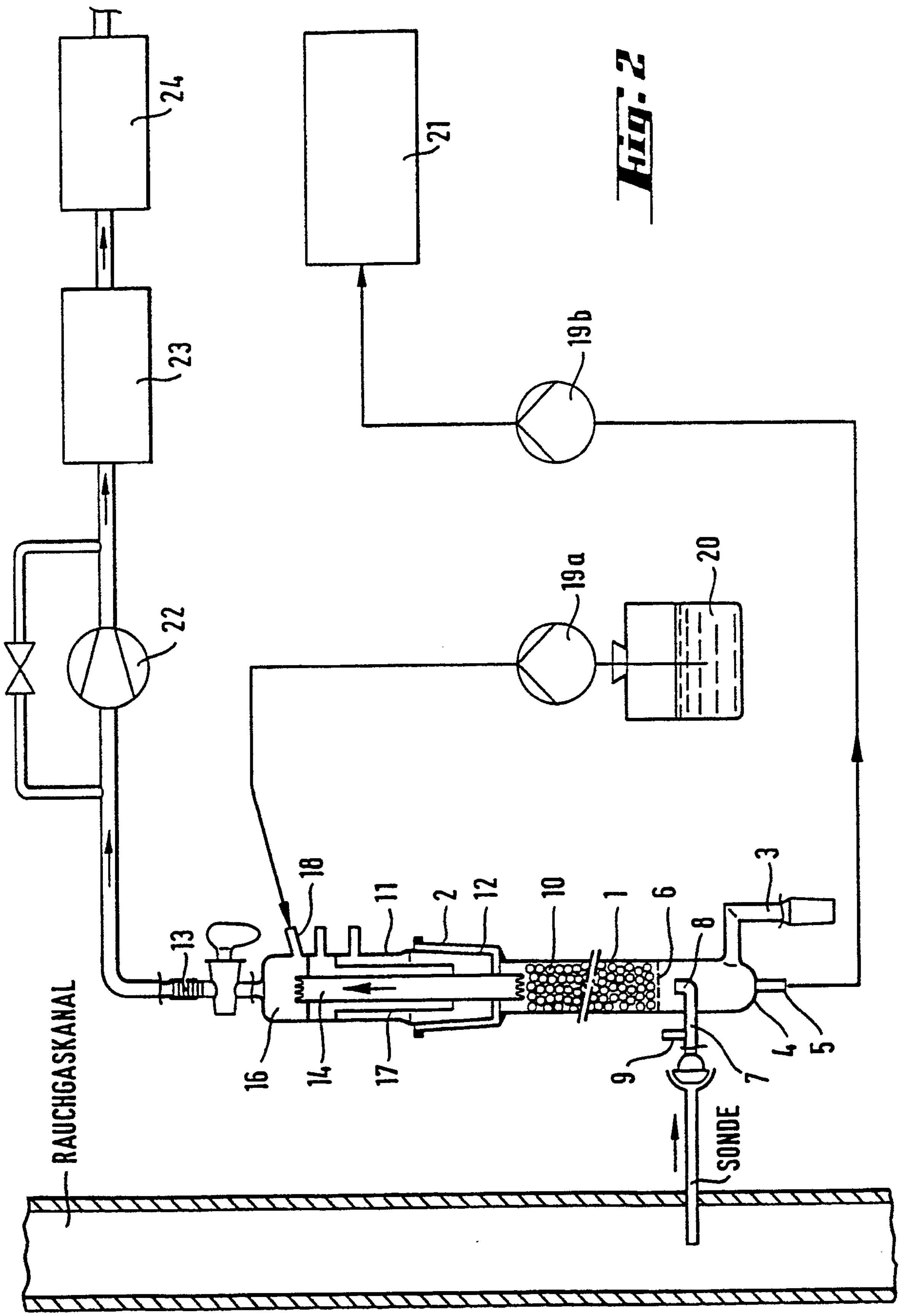
RAUCHGASKANAL
SONDE
1
2
3
4
5
6
7
8
9
10
11
12
13
14
16
17
18
19a
19b
20
21
22
23
24

Fig. 2